# EUROPÄISCHE PATENTSCHRIFT

(11) **EP 2 651 816 B1**
(45) Veröffentlichungstag und Bekanntmachung des Hinweises auf die Patenterteilung: **25.02.2015**
(21) Anmeldenummer: 11796632.5
(22) Anmeldetag: 13.12.2011
(51) Int. Cl.: A61K 8/04, A61Q 5/04, A61Q 5/08, A61Q 5/10, D06L 3/02, C01B 15/08, C01B 15/037, C01B 15/12, A61K 8/22, C07D 213/89, C11D 3/28, C11D 3/32, C11D 3/39, C11D 7/32, A61K 8/49

(54) **ZUSAMMENSETZUNGEN ENTHALTEND WASSERSTOFFPEROXID ODER WASSERSTOFFPEROXID FREISETZENDE SUBSTANZEN**
COMPOSITIONS CONTAINING HYDROGEN PEROXIDE OR SUBSTANCES RELEASING HYDROGEN PEROXIDE
COMPOSITIONS CONTENANT DU PEROXYDE D'HYDROGÈNE OU DES SUBSTANCES LIBÉRANT DU PEROXYDE D'HYDROGÈNE

(30) Priorität: 17.12.2010 DE 102010054866
(43) Veröffentlichungstag der Anmeldung: 23.10.2013
(73) Patentinhaber: Clariant Finance (BVI) Limited, Road Town, Tortola (VG)
(72) Erfinder: KLUG, Peter, 63762 Großostheim (DE); PILZ, Maurice, Frederic, 60329 Frankfurt am Main (DE); BACK, Ute, 63825 Blankenbach (DE)
(74) Vertreter: Paczkowski, Marcus
(86) Internationale Anmeldenummer: PCT/EP2011/006277
(87) Internationale Veröffentlichungsnummer: WO 2012/079739

(56) Entgegenhaltungen:
- EP-A1- 1 347 736
- WO-A2-02/051961
- FR-A1- 2 804 863
- US-A- 5 206 385

## Beschreibung

Die vorliegende Erfindung betrifft Zusammensetzungen enthaltend Wasserstoffperoxid oder Wasserstoffperoxid freisetzende Substanzen.

Wasserhaltige Zusammensetzungen enthaltend Wasserstoffperoxid werden in verschiedenen Anwendungen benutzt. Sie werden in kosmetischen Mitteln z. B. als Bleichzusammensetzung für Haar, als Entwicklerkomponente in Haarfärbemitteln, aber auch als Komponente zur Haarfixierung in Dauerwellformulierungen verwendet. Weitere Anwendungen sind z. B. Zahnbleichzusammensetzungen. Auch in der industriellen Reinigung und in der Haushaltsreinigung und in der Textilbleiche sind saure, wasserstoffperoxidhaltige Zusammensetzungen in Reinigerformulierungen vertreten.

Allerdings ist die Stabilität des Wasserstoffperoxids oder der Wasserstoffperoxid freisetzenden Substanzen in Zusammensetzungen, die keine verdickenden Polymere enthalten, oftmals unbefriedigend.

In der Literatur werden verschiedene Stabilisatoren für Wasserstoffperoxid genannt, z. B. beschreibt die EP 1 347 736 oxidative Zusammensetzungen zur Haarbehandlung, die Stabilisatoren auf Basis Pyrophosphat, Stannaten, Phenacetin oder Oxychinolin oder deren Kombinationen enthalten. Diese Zusammensetzungen enthalten jedoch auch Polymere mit verdickenden Eigenschaften.

WO 02/051961 A offenbart eine Zusammensetzung enthaltend Wasserstoffperoxid und eine stabilisierende Substanz, d.h. Pyridinon gemäß der Formel 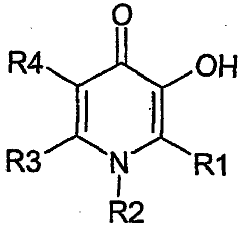 worin R1, R2, R3 und R4 aus der Gruppe bestehend aus H und jeder beliebigen organischen Seitenkette ausgewählt sind.

Es bestand deshalb die Aufgabe, langfristig lagerstabile Zusammensetzungen bereit zu stellen, die Wasserstoffperoxid und/oder Wasserstoffperoxid freisetzende Substanzen enthalten, aber keine Polymere mit verdickenden Eigenschaften.

Überraschend wurde nun gefunden, dass diese Aufgabe gelöst wird, indem Hydroxypyridone oder deren Salze in wässrige Zusammensetzungen eingearbeitet werden, die zwar eine oder mehrere Substanzen ausgewählt aus der Gruppe bestehend aus Wasserstoffperoxid und Wasserstoffperoxid freisetzenden Substanzen enthalten aber keine Polymere mit verdickenden Eigenschaften.

Gegenstand der Erfindung sind somit Zusammensetzungen enthaltend
a) eine oder mehrere Substanzen ausgewählt aus der Gruppe bestehend aus Wasserstoffperoxid und Wasserstoffperoxid freisetzenden Substanzen,
b) Wasser,
d) eine oder mehrere Substanzen ausgewählt aus der Gruppe bestehend aus Hydroxypyridonen und deren Salzen.
dadurch gekennzeichnet, dass sie kein Polymer mit verdickenden Eigenschaften enthalten.

Durch die erfindungsgemäßen Zusammensetzungen sind nun auch niedrigviskose Zusammensetzungen zugänglich, die Wasserstoffperoxid und/oder Wasserstoffperoxid freisetzende Substanzen mit erhöhter Stabilität enthalten. Niedrigviskose Zusammensetzungen werden z. B. im Bereich der Reinigerformulierungen oder auch der Textilbleiche benötigt. Durch die erhöhte Stabilität des Wasserstoffperoxids und/oder der Wasserstoffperoxid freisetzenden Substanzen in diesen Zusammensetzungen kann eine verbesserte Reinigungs- oder Bleichleistung erzielt werden.

Vorzugsweise ist die eine oder sind die mehreren Substanzen der Komponente a) ausgewählt aus der Gruppe bestehend aus Wasserstoffperoxid, Harnstoffperoxid, Perboraten, Persulfaten und Mischungen davon. Besonders bevorzugt ist die Substanz der Komponente a) Wasserstoffperoxid.

Die eine oder die mehreren Substanzen ausgewählt aus der Gruppe bestehend aus Wasserstoffperoxid und Wasserstoffperoxid freisetzenden Substanzen der Komponente a) sind vorzugsweise in Mengen von 0,5 bis 20 Gew.-%, besonders bevorzugt in Mengen von 1 bis 10 Gew.-%, insbesondere bevorzugt in Mengen von 1,5 bis 7 Gew.-% und außerordentlich bevorzugt in Mengen von 2 bis 7 Gew.-% in den erfindungsgemäßen Zusammensetzungen enthalten, bezogen auf das Gesamtgewicht der Zusammensetzungen. Hierunter wiederum bevorzugt ist die Substanz der Komponente a) Wasserstoffperoxid, welches vorzugsweise in Mengen von 0,5 bis 20 Gew.-%, besonders bevorzugt in Mengen von 1 bis 10 Gew.-%, insbesondere bevorzugt in Mengen von 1,5 bis 7 Gew.-% und außerordentlich bevorzugt in Mengen von 2 bis 7 Gew.-% in den erfindungsgemäßen Zusammensetzungen enthalten ist, bezogen auf das Gesamtgewicht der Zusammensetzungen.

In einer bevorzugten Ausführungsform der Erfindung ist das Wasser (Komponente b)) in einer Menge von 40 Gew.-% oder mehr und vorzugsweise in einer Menge von 50 Gew.-% oder mehr in den erfindungsgemäßen Zusammensetzungen enthalten, bezogen auf das Gesamtgewicht der Zusammensetzungen.

Unter Polymeren mit verdickenden Eigenschaften werden im Rahmen der vorliegenden Erfindung vorzugsweise polymere Materialien mit einem Molekulargewicht oberhalb 5 000 g/mol verstanden, die geeignet sind, eine Zusammensetzung enthaltend eine oder mehrere Substanzen ausgewählt aus der Gruppe bestehend aus Wasserstoffperoxid und Wasserstoffperoxid freisetzenden Substanzen bei Einsatzmengen des Polymers von 30 Gew.-% oder weniger, vorzugsweise von 10 Gew.-% oder weniger, besonders bevorzugt von 6 Gew.-% oder weniger und insbesondere bevorzugt von 3 Gew.-% oder weniger, bezogen auf das Gesamtgewicht der Zusammensetzung, signifikant zu verdicken. Diese Verdickung ist vorzugsweise derart, dass die Viskosität der Zusammensetzung in mPa·s bei 20 °C mit den oben genannten Mengen der Polymere um 30 % oder mehr erhöht wird.

Die Viskosität der erfindungsgemäßen Zusammensetzungen sind vorzugsweise ≤ 2 000 mPa·s bei 20 °C, besonders bevorzugt ≤ 1 000 mPa·s bei 20 °C und insbesondere bevorzugt ≤ 500 mPa·s bei 20 °C. Die Viskositäten werden an den erfindungsgemäßen Zusammensetzungen selbst mit einem Brookfield-Viskosimeter Model DV II bei 20 Umdrehungen / Minute und 20 °C gemessen. Es werden die Spindeln aus dem Spindelset RV verwendet. Unter diesen Messbedingungen wird Spindel 1 für Viskositäten von maximal 500 mPa·s, Spindel 2 für Viskositäten von maximal 1 000 mPa·s und Spindel 3 für Viskositäten von maximal 5 000 mPa·s gewählt.

Vorzugsweise enthalten die erfindungsgemäßen Zusammensetzungen keine Polymere.

Vorzugsweise ist die eine oder sind die mehreren Substanzen der Komponente d) ausgewählt aus Verbindungen der Formel (I) und deren Salzen 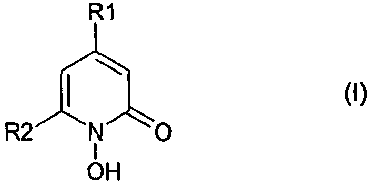 worin R1 H oder ein C₁-C₄ Alkylrest und R2 H, ein unsubstituierter oder mit Halogen substituierter, verzweigter oder unverzweigter C₁-C₂₀-Alkylrest, ein unsubstituierter oder mit Halogen substituierter C₅-C₈ Cycloalkylrest, ein unsubstituierter oder mit Halogen substituierter C₆-C₁₀ Arylrest oder ein unsubstituierter oder mit Halogen substituierter, verzweigter oder unverzweigter C₇-C₂₀ Aralkylrest ist.

Vorzugsweise sind die Reste R2 nicht mit Halogen substituiert.

In einer bevorzugten Ausführungsform der Erfindung liegt die eine oder liegen die mehreren Verbindungen der Komponente d) in Form der Säure (Verbindungen der Formel (I)) oder in Form ihrer Alkali-, Erdalkali- oder Aminsalze oder ihrer Salze mit polymeren Gegenionen in den erfindungsgemäßen Zusammensetzungen vor. In der einen oder in den mehreren Verbindungen der Formel (I) oder in ihren Salzen ist R1 vorzugsweise Methyl und R2 vorzugsweise Cyclohexyl oder 2,4,4-Trimethylpentyl.

Besonders bevorzugt liegen die Verbindungen der Formel (I) in Form ihrer Alkanolaminsalze und insbesondere bevorzugt in Form ihrer Monoethanolaminsalze vor. Beispiele für derartige Salze sind in DE 2234009 erwähnt.

Besonders bevorzugt sind dabei 4-Methyl-6-(2,4,4-trimethylpentyl)-1-hydroxy-2-pyridon, das Monoethanolaminsalz von 4-Methyl-6-(2,4,4-trimethylpentyl)-1-hydroxy-2-pyridon (Octopirox^{®}, Clariant) sowie 4-Methyl-6-(cyclohexyl)-1-hydroxy-2-pyridon und das Monoethanolaminsalz von 4-Methyl-6-(cyclohexyl)-1-hydroxy-2-pyridon (Ciclopirox^{®}, Sanofi-Aventis).

Diese Substanzen können anhand literaturbekannter Verfahren erhalten werden, vergleiche hierzu die in DE 2234009 genannten Referenzen.

In den erfindungsgemäßen Zusammensetzungen ist die eine oder sind die mehreren Substanzen der Komponente d) in Mengen von vorzugsweise 0,1 bis 20 000 ppm (0,00001 bis 2 Gew.-%), besonders bevorzugt in Mengen von 0,5 bis 1 000 ppm (0,00005 bis 0,1 Gew.-%) und insbesondere bevorzugt in Mengen von 0,5 bis 100 ppm (0,00005 bis 0,01 Gew.-%) enthalten, bezogen auf das Gesamtgewicht der Zusammensetzungen.

Die Hydroxypyridone können in den erfindungsgemäßen Zusammensetzungen mit weiteren Stabilisatoren kombiniert werden. Weitere geeignete Stabilisatoren sind z. B. Polyphosphate oder deren Alkali- oder Erdalkalimetallsalze, Alkali- oder Erdalkalistannate, Phenacetin und seine Säuresalze sowie Oxychinolin und seine Säuresalze. Im Allgemeinen enthalten die lieferfertigen Wasserstoffperoxidlösungen bereits Stabilisatoren, bevorzugt in Form von Polyphosphaten.

Die erfindungsgemäßen Zusammensetzungen können eingesetzt werden als Bleichzusammensetzungen für Haar oder Zähne, als Bleichkomponente oder Entwickler für oxidative Haarfarben oder als Fixierkomponente für Dauerwellformulierungen und als Haushaltsreiniger. Darüber hinaus können die erfindungsgemäßen Zusammensetzungen z. B. als Vorwaschsprays, Fleckentferner, Oberflächenreiniger, oder Toilettenreiniger Verwendung finden. Eine weitere mögliche Verwendung sind auch Haarfärbezusammensetzungen zur Verhinderung von Bleichschäden bei der Haarfärbung.

In einer weiteren bevorzugten Ausführungsform der Erfindung sind die erfindungsgemäßen Zusammensetzungen Emulsionen. Bei den Emulsionen handelt es sich bevorzugt um Öl-in-Wasser-Emulsionen oder Mikroemulsionen.

Der nichtwässrige Anteil dieser Emulsionen, der sich weitgehend aus dem Emulgator und dem Ölkörper zusammensetzt, liegt üblicherweise bei 0,5 bis 20,0 Gew.-% und vorzugsweise bei 1,0 bis 10,0 Gew.-%, bezogen auf das Gesamtgewicht der Emulsionen. Daraus folgt, dass die Emulsionen 80,0 bis 99,5 Gew.-% und vorzugsweise 90,0 bis 99,0 Gew.-% der wässrigen Phase enthalten können, bezogen auf das Gesamtgewicht der Emulsionen.

Die erfindungsgemäßen Zusammensetzungen können auch anionische, kationische, nichtionische, ampholytische Tenside und/oder Betaintenside enthalten.

Die Gesamtmenge der in den erfindungsgemäßen Zusammensetzungen eingesetzten Tenside beträgt, bezogen auf das Gesamtgewicht der Zusammensetzungen, vorzugsweise von 0,1 bis 20 Gew.-%, besonders bevorzugt von 0,5 bis 10,0 Gew.-% und insbesondere bevorzugt von 1,0 bis 5,0 Gew.-%.

Als anionische Tenside bevorzugt sind (C₁₀-C₂₂)-Alkyl- und Alkylencarboxylate, Alkylethercarboxylate, Fettalkoholsulfate, Fettalkoholethersulfate, Alkylamidsulfate und -sulfonate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den erfindungsgemäßen Zusammensetzungen beträgt bevorzugt von 0,05 bis 20,0 Gew.-%, besonders bevorzugt von 0,5 bis 10,0 Gew.-% und insbesondere bevorzugt von 1,0 bis 5,0 Gew.-%, bezogen auf das Gesamtgewicht der Zusammensetzungen.

Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyl-dimethylammoniumchlorid oder -bromid; (C₈-C₂₂)-Alkyl-dimethyl-ethylammoniumchlorid oder -bromid; (C₈-C₂₂)-Alkyl-trimethylammoniumchlorid oder -bromid, vorzugsweise Cetyltrimethylammoniumchlorid oder -bromid und (C₈-C₂₂)-Alkyl-trimethylammoniumchlorid oder -bromid; (C₁₀-C₂₄)-Alkyl-dimethylbenzyl-ammoniumchlorid oder -bromid, vorzugsweise (C₁₂-C₁₄)-Alkyl-dimethylbenzyl-ammoniumchlorid, (C₈-C₂₂)-Alkyl-dimethyl-hydroxyethylammoniumchlorid, -phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat, sowie Esterquats auf Basis von C₈-C₂₂ Alkanoylestern von Triethanolamin oder Methyldiethanolamin.

Die Menge der kationischen Tenside in den erfindungsgemäßen Zusammensetzungen beträgt bevorzugt von 0,1 bis 10,0 Gew.-%, besonders bevorzugt von 0,5 bis 7,0 Gew.-% und insbesondere bevorzugt von 1,0 bis 5,0 Gew.-%, bezogen auf das Gesamtgewicht der Zusammensetzungen.

Als nichtionische Tenside bevorzugt sind beispielsweise Fettsäurealkanolamide; Saccharoseester; Sorbitester und Sorbitanester sowie C₈-C₂₂-Alkylpolyglucoside.

Die Menge der nichtionischen Tenside in den erfindungsgemäßen Zusammensetzungen (z. B. im Falle von Rinse-off-Produkten) liegt bevorzugt im Bereich von 0,05 bis 20,0 Gew.-%, besonders bevorzugt von 0,1 bis 10,0 Gew.-% und insbesondere bevorzugt von 0,5 bis 5,0 Gew.-%, bezogen auf das Gesamtgewicht der Zusammensetzungen.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimethylbetaine.

Die Menge der amphoteren Tenside und/oder Betaintenside in den erfindungsgemäßen Zusammensetzungen beträgt bevorzugt von 0,5 bis 20,0 Gew.-% und besonders bevorzugt von 1,0 bis 10,0 Gew.-%, bezogen auf das Gesamtgewicht der Zusammensetzungen.

Bevorzugte Tenside sind Laurylsulfat, Laurethsulfat, Cocoamidopropylbetain, Alkylbetaine wie Coco-Betain, Aminoxide, Natriumcocoylglutamat und Lauroamphoacetat.

Die erfindungsgemäßen Zusammensetzungen können als weitere Hilfs- und Zusatzstoffe z. B. Ölkörper, Wachse, Emulgatoren, Co-Emulgatoren, Solubilisatoren, Überfettungsmittel, Rückfetter, antimikrobielle Wirkstoffe, Feuchthaltemittel, Lösemittel, Farbstoffe, Duftstoffe, Perlglanzmittel und/oder Trübungsmittel enthalten.

Die Ölkörper können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel", Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318. Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈-₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Buty1-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®}der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat. Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe, Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®} S), Ozokerit und Ceresin.

Die erfindungsgemäßen Zusammensetzungen können Wachse, beispielsweise Paraffinwachse, Mikrowachse und Ozokerite, Bienenwachs und ihre Teilfraktionen, Bienenwachsderivate, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht:
Fettalkohole mit 8 bis 22 C-Atomen, Glycerinmono- und -diester und Sorbitanmono- und -diester von gesättigten und ungesättigten Fettsäuren mit 6 bis 22 Kohlenstoffatomen; Polyolester; Fettsäureamide; Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats.

An amphoteren Emulgatoren stehen vorzugsweise zur Verfügung Alkylaminoalkylcarbonsäuren, Betaine, Sulfobetaine und Imidazolinderivate.

Besonders vorteilhafte Coemulgatoren sind Glycerylmonostearat, Glycerylmonooleat, Diglycerylmonostearat, Glycerylisostearat, Glycoldistearat, Sorbitanmonoisostearat, Sorbitanstearat, Sorbitanoleat, Saccharosedistearat, Lecithin, Cetylalkohol, Stearylalkohol, Behenylalkohol und Isobehenylalkohol.

Die erfindungsgemäßen Zusammensetzungen können einen oder mehrere der Emulgatoren, Co-Emulgatoren oder Solubilisatoren in Mengen von 0,1 bis 20,0 Gew.-%, vorzugsweise von 1,0 bis 15,0 Gew.-% und besonders bevorzugt von 3,0 bis 10,0 Gew.-%, bezogen auf das Gesamtgewicht der Zusammensetzungen, enthalten.

Als Oberfettungsmittel können vorzugsweise Lanolin und Lecithin, nicht ethoxylierte Lanolin- und Lecithinderivate oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden, bezogen auf das Gesamtgewicht der Zusammensetzungen.

An antimikrobiellen Wirkstoffen können Cetyltrimethylammoniumchlorid, Cetylpyridiniumchlorid, Benzethoniumchlorid, Diisobutylethoxyethyldimethylbenzylammoniumchlorid, Natriumaluminiumchlorohydroxylactat, Triethylcitrat, 2,4,4'-Trichloro-2'-hydroxydiphenylether (Triclosan), 3,4,4'-Trichlorocarbanilid (Triclocarban), Diaminoalkylamid, beispielsweise L-Lysinhexadecylamid, Citratschwermetallsalze, Salicylate, Pyrithione und deren Schwermetallsalze, insbesondere Zinkpyrithion, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Selendisulfid, Iodopropynylbutylcarbamat, Methylchloroisothiazolinon, Methylisothiazolinon, Methyldibromo Glutaronitril, AgCl, Chloroxylenol, Natriumbenzoat, sowie Phenoxyethanol, Phenoxyisopropanol, Parabene, bevorzugt Butyl-, Ethyl-, Methyl- und Propylparaben, sowie deren Na-Salze, Pentandiol, 1,2-Hexandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat eingesetzt werden.

Die erfindungsgemäßen Zusammensetzungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf das Gesamtgewicht der Zusammensetzungen.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), DL-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.-% und besonders bevorzugt von 0,5 bis 5,0 Gew.-%, bezogen auf das Gesamtgewicht der Zusammensetzungen.

Zusätzlich können die erfindungsgemäßen Zusammensetzungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin, 1,2 Propandiol und 1,3 Propandiol und Mischungen aus den genannten Alkoholen eingesetzt. Weitere geeignete Lösungsmittel sind beispielsweise Triacetin (Glycerintriacetat) und 1-Methoxy-2-propanol.

Die erfindungsgemäßen Zusammensetzungen können Farbstoffe- und -pigmente, sowohl organische als auch anorganische Farbstoffe, enthalten, die aus der entsprechenden Positivliste der Kosmetikverordnung bzw. der EG-Liste kosmetischer Färbemittel ausgewählt sind.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxynaphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| Trans-ß-Apo-8'-Carotinaldehyd (C₃₀) | 40820 | orange |
| Trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenrylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthloindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| Trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z. B. Paprikaextrakte, β-Carotin und Cochenille.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Sofern die erfindungsgemäßen Zusammensetzungen perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zusammensetzungen enthalten, bezogen auf das Gesamtgewicht der Zusammensetzungen.

Als Säuren oder Laugen zur pH-Wert Einstellung werden vorzugsweise Mineralsäüren, insbesondere HCl, anorganische Basen, insbesondere NaOH oder KOH, und organische Säuren, insbesondere Zitronensäure, verwendet. Vorzugsweise weisen die erfindungsgemäßen Zusammensetzungen einen pH-Wert von 2 bis 11, besonders bevorzugt von 7 bis 11, insbesondere bevorzugt von 8 bis 11 und außerordentlich bevorzugt von 8,5 bis 11, auf.

In einer weiteren bevorzugten Ausführungsform der Erfindung handelt es sich bei den erfindungsgemäßen Zusammensetzungen um Zusammensetzungen zum Bleichen und/oder Färben von Haaren oder um eine Dauerwellformulierung.

In einer weiteren bevorzugten Ausführungsform der Erfindung handelt es sich bei den erfindungsgemäßen Zusammensetzungen um oxidative Reinigerformulierungen.

In einer weiteren bevorzugten Ausführungsform der Erfindung handelt es sich bei den erfindungsgemäßen Zusammensetzungen um Zusammensetzungen zum oxidativen Bleichen von Fasern oder Textilien.

Die erfindungsgemäßen Zusammensetzungen sind in vorteilhafter Weise zur Reduzierung der Haarschädigung in Haarfärbeformulierungen geeignet.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

### Versuchsbeispiele:

### Beispiel 1:

Wasserstoffperoxidlösung von Solvay (35 Gew.-% in Wasser) bzw. von Merck (35 Gew.-% in Wasser) wurde auf einen Wasserstoffperoxidgehalt von etwa 6,0 Gew.-% mit vollentsalztem Wasser verdünnt und mit Natronlauge (20 Gew.-%) auf einen pH-Wert von 9,0 eingestellt. Weitere Lösungen wurden jeweils mit 8 ppm 4-Methyl-6-(2,4,4 trimethylpentyl)-1-hydroxy-2-pyridon (Additiv A, gelöst in Propylenglykol) additivert. Die Lösungen wurden bei Raumtemperatur (20 °C) und 40 °C 1 Woche gelagert und der Wasserstoffperoxidgehalt vor und nach Lagerung gemessen (siehe Tabelle 1).

**Tabelle 1 Ergebnisse der Messung des Wasserstoffperoxidgehalts**

| Wasserstoffperoxid | Additiv A | Wasserstoff-peroxidgehalt sofort | Wasserstoffperoxidgehalt nach 1 Woche 20 °C | Wasserstoff-peroxidgehalt nach 1 Woche 40 °C |
|---|---|---|---|---|
| | | [Gew.-%] | [Gew.-%] | [Gew.-%] |
| Solvay (35 Gew.-%) | Nein | 6,3 | 4,8 | < 0,1 |
| Solvay (35 Gew.-%) | Ja | 6,0 | 6,1 | 5,9 |
| Merck (35 Gew.-%) | Nein | 5,9 | 3,9 | 2,0 |
| Merck (35 Gew.-%) | ja | 6,1 | 6,1 | 5,8 |

### Beispiel 2:

Wasserstoffperoxidlösung von Solvay (35 Gew.-% in Wasser) bzw. von Merck (35 Gew.-% in Wasser), eine Lösung von Sodium C₁₄₋₁₇ Alkyl sec. Sulfonate (Hostapur^{®} SAS 30) und vollentsalztes Wasser wurden so gemischt, dass ein Wasserstoffperoxidgehalt von etwa 6,0 Gew.-% sowie ein Gehalt an Sodium C₁₄₋₁₇ Alkyl sec. Sulfonate von 5,0 Gew.-% resultierte. Danach wurde mit Natronlauge (20 Gew.-%) auf einen pH-Wert von 9,0 eingestellt. Weitere Lösungen wurden jeweils mit 7 ppm 4-Methyl-6-(2,4,4 trimethylpentyl)-1-hydroxy-2-pyridon (Additiv A, gelöst in Propylenglykol) additivert. Die Lösungen wurden bei Raumtemperatur und 40 °C 1 Woche gelagert und der Wasserstoffperoxidgehalt vor und nach Lagerung gemessen (siehe Tabelle 2).

**Tabelle 2 Ergebnisse der Messung des Wasserstoffperoxidgehalts**

| Wasserstoffperoxid | Additiv A | Wasserstoffperoxidgehalt sofort | Wasserstoff-peroxidgehalt nach 1 Woche 20 °C | Wasserstoff-peroxidgehalt nach 1 Woche 40 °C |
|---|---|---|---|---|
| | | [Gew.-%] | [Gew.-%] | [Gew.-%] |
| Solvay (35 Gew.-%) | Nein | 6,1 | 3,7 | 0,1 |
| Solvay (35 Gew.-%) | Ja | 6,0 | 6,1 | 5,8 |
| Merck (35 Gew.-%) | Nein | 5,9 | 4,2 | 1,4 |
| Merck (35 Gew.-%) | Ja | 6,0 | 6,1 | 5,2 |

Die Ergebnisse der Beispiele 1 und 2 zeigen, dass 4-Methyl-6-(2,4,4 trimethylpentyl)-1-hydroxy-2-pyridon sowohl bei Raumtemperatur als auch bei 40 °C die Lagerstabilität von Wasserstoffperoxidlösungen bei hohem pH-Wert signifikant steigern kann.

### Formulierungsbeispiele:

Formulierungsbeispiel 1: Vorwaschspray für Wäsche

| Inhaltsstoff | Gew.-% |
|---|---|
| Wasserstoffperoxid (35 Gew.-%ig, wässrig) | 10,0 |
| Decyl Glucoside (50 Gew.-%ige wässrige Lösung) | 5,0 |
| Coco Glucoside (50 Gew.-%ige wässrige Lösung) | 2,0 |
| Hostapur® SAS 60 (Sodium C14-17 Alkyl Sec Sulfonate) | 5,0 |
| 4-Methyl-6-(2,4,4-trimethylpentyl)-1-hydroxy-2-pyridon, Monoethanolaminsalz | 0,0005 |
| Propylenglykol | 1,0 |
| Wasser | ad 100 |

### Herstellung:

4-Methyl-6-(2,4,4-trimethylpentyl)-1-hydroxy-2-pyridon Monoethanolaminsalz (Octopirox^{®}) wird in Propylenglykol gelöst. Die Tenside werden in Wasser gelöst, die Wasserstoffperoxidlösung sowie die Propylenglykollösung eingetragen und homogenisiert. Das Produkt wird auf pH = 4 eingestellt.

### Formulierungsbeispiel 2: Fleckentferner

| Inhaltsstoff | Gew.-% |
|---|---|
| Wasserstoffperoxid (35 Gew.-%ig, wässrig) | 10,0 |
| Sodium C12-C14 Olefin Sulfonate | 5,0 |
| 4-Methyl-6-(cyclohexyl)-1-hydroxy-2-pyridon | 0,0005 |
| Propylenglykol | 1,0 |
| Wasser | ad 100 |

### Herstellung:

4-Methyl-6-(cyclohexyl)-1-hydroxy-2-pyridon wird in Propylenglykol gelöst. Das Tensid wird in Wasser gelöst, die Wasserstoffperoxidlösung sowie die Propylenglykollösung eingetragen und homogenisiert. Das Produkt wird auf pH = 7 eingestellt.

### Formulierungsbeispiel 3: Fleckentferner

| Inhaltsstoff | Gew.-% |
|---|---|
| Wasserstoffperoxid (35 Gew.-%ig, wässrig) | 12,0 |
| Lauryl Glucoside (50 Gew.-%ige wässrige Lösung) | 7,0 |
| 4-Methyl-6-(2,4,4-trimethylpentyl)-1-hydroxy-2-pyridon | 0,0005 |
| Propylenglykol | 1,0 |
| MEA-Dodecylbenzolsulfonate | 6,0 |
| Wasser | ad 100 |

### Herstellung:

4-Methyl-6-(2,4,4-trimethylpentyl)-1-hydroxy-2-pyridon wird in Propylenglykol gelöst.

Die Tenside werden in Wasser gelöst, die Wasserstoffperoxidlösung sowie die Propylenglykollösung eingetragen und homogenisiert. Das Produkt wird auf pH = 4,7 eingestellt.

## Patentansprüche

1. Zusammensetzung enthaltend
a) eine oder mehrere Substanzen ausgewählt aus der Gruppe bestehend aus Wasserstoffperoxid und Wasserstoffperoxid freisetzenden Substanzen,
b) Wasser,
d) eine oder mehrere Substanzen ausgewählt aus Verbindungen der Formel (I) und deren Salzen 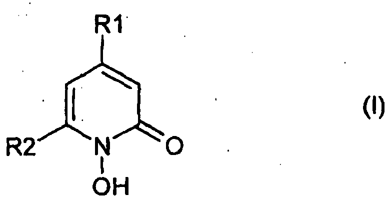 worin R1 H oder ein C₁-C₄ Alkylrest und R2 H, ein unsubstituierter oder mit Halogen substituierter, verzweigter oder unverzweigter C₁-C₂₀-Alkylrest, ein unsubstituierter oder mit Halogen substituierter C₅-C₈ Cycloalkylrest, ein unsubstituierter oder mit Halogen substituierter C₆-C₁₀ Arylrest oder ein unsubstituierter oder mit Halogen substituierter, verzweigter oder unverzweigter C₇-C₂₀ Aralkylrest ist,
**dadurch gekennzeichnet, dass** sie kein Polymer mit verdickenden Eigenschaften enthält.

2. Zusammensetzung nach Anspruch 1, **dadurch gekennzeichnet, dass** die eine oder die mehreren Substanzen der Komponente a) ausgewählt sind aus der Gruppe bestehend aus Wasserstoffperoxid, Harnstoffperoxid, Perboraten, Persulfaten und Mischungen davon.

3. Zusammensetzung nach Anspruch 1 oder 2, **dadurch gekennzeichnet, dass** die Substanz der Komponente a) Wasserstoffperoxid ist und das Wasserstoffperoxid in Mengen von 0,5 bis 20 Gew.-% in der Zusammensetzung enthalten ist, bezogen auf das Gesamtgewicht der Zusammensetzung.

4. Zusammensetzung nach einem oder mehreren der Ansprüche 1 bis 3, **dadurch gekennzeichnet, dass** die eine oder die mehreren Verbindungen der Komponente d) in Form der Säure oder in Form ihrer Alkali-, Erdalkali- oder Aminsalze oder in Form ihrer Salze mit polymeren Gegenionen vorliegen.

5. Zusammensetzung nach einem oder mehreren der Ansprüche 1 bis 4, **dadurch gekennzeichnet, dass** in der einen oder in den mehreren Verbindungen der Formel (I) oder in ihren Salzen R1 Methyl ist und R2 Cyclohexyl oder 2,4,4-Trimethylpentyl ist.

6. Zusammensetzung nach einem oder mehreren der Ansprüche 1 bis 5, **dadurch gekennzeichnet, dass** sie die eine oder die mehreren Substanzen der Komponente d) in Mengen von 0,1 ppm bis 2 Gew.-% enthält, bezogen auf das Gesamtgewicht der Zusammensetzung.

7. Zusammensetzung nach Anspruch 6, **dadurch gekennzeichnet, dass** sie die eine oder die mehreren Substanzen der Komponente d) in Mengen von 0,5 bis 100 ppm enthält, bezogen auf das Gesamtgewicht der Zusammensetzung.

8. Zusammensetzung nach einem oder mehreren der Ansprüche 1 bis 7, **dadurch gekennzeichnet, dass** sie einen pH-Wert von 2 bis 11 aufweist.

9. Zusammensetzung nach Anspruch 8, **dadurch gekennzeichnet, dass** sie einen pH-Wert von 7 bis 11 aufweist.

10. Zusammensetzung nach einem oder mehreren der Ansprüche 1 bis 9, **dadurch gekennzeichnet, dass** sie eine Zusammensetzung zum Bleichen und/oder Färben von Haaren oder eine Dauerwellformulierung ist.

11. Zusammensetzung nach einem oder mehreren der Ansprüche 1 bis 9, **dadurch gekennzeichnet, dass** sie eine oxidative Reinigerformulierung ist.

12. Zusammensetzung nach einem oder mehreren der Ansprüche 1 bis 9, **dadurch gekennzeichnet, dass** sie eine Zusammensetzung zum oxidativen Bleichen von Fasern oder Textilien ist.

## Claims

1. A composition comprising
a) one or more substances selected from the group consisting of hydrogen peroxide and hydrogen peroxide-releasing substances,
b) water,
d) one or more substances selected from compounds of the formula (I) and salts thereof 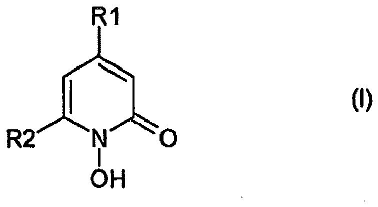 in which R1 is H or a C₁-C₄ alkyl radical and R2 is H, an unsubstituted or halogen-substituted, branched or unbranched C₁-C₂₀ alkyl radical, an unsubstituted or halogen-substituted C₅-C₈ cycloalkyl radical, an unsubstituted or halogen-substituted C₆-C₁₀ aryl radical or an unsubstituted or halogen-substituted, branched or unbranched C₇-C₂₀ aralkyl radical,
wherein the composition does not comprise any polymer having thickening properties.

2. The composition as claimed in claim 1, wherein the one or more substances of component a) are selected from the group consisting of hydrogen peroxide, urea peroxide, perborates, persulfates and mixtures thereof.

3. The composition as claimed in claim 1 or 2, wherein the substance of component a) is hydrogen peroxide and the hydrogen peroxide is present in the composition in amounts of 0.5 to 20% by weight based on the total weight of the composition.

4. The composition as claimed in one or more of claims 1 to 3, wherein the one or more compounds of component d) are present in the form of the acid or in the form of the alkali metal, alkaline earth metal or amine salts thereof or salts thereof with polymeric counterions.

5. The composition as claimed in one or more of claims 1 to 4, wherein, in the one or more compounds of the formula (I) or in the salts thereof, R1 is methyl and R2 is cyclohexyl or 2,4,4-trimethylpentyl.

6. The composition as claimed in one or more of claims 1 to 5, which comprises the one or more substances of component d) in amounts of 0.1 ppm to 2% by weight, based on the total weight of the composition.

7. The composition as claimed in claim 6, which comprises the one or more substances of component d) in amounts of 0.5 to 100 ppm, based on the total weight of the composition.

8. The composition as claimed in one or more of claims 1 to 7, which has a pH of 2 to 11.

9. The composition as claimed in claim 8, which has a pH of 7 to 11.

10. The composition as claimed in one or more of claims 1 to 9, which is a composition for bleaching and/or coloring of hair or a permanent wave formulation.

11. The composition as claimed in one or more of claims 1 to 9, which is an oxidative cleaner formulation.

12. The composition as claimed in one or more of claims 1 to 9, which is a composition for oxidative bleaching of fibers or textiles.

## Revendications

1. Composition contenant
a) une ou plusieurs substances choisies dans le groupe constitué par le peroxyde d'hydrogène et des substances libérant du peroxyde d'hydrogène,
b) de l'eau,
c) une ou plusieurs substances choisies parmi des composés de formule (I) et leurs sels 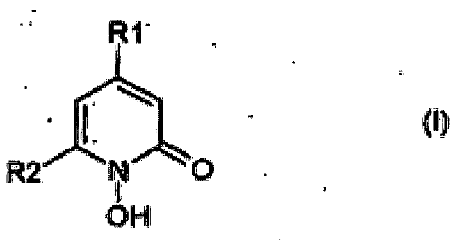 dans laquelle R1 est H ou un radical alkyle en C₁-C₄ et R2 est H, un radical alkyle en C₁-C₂₀ ramifié ou non ramifié, non substitué ou substitué par halogéno, un radical cycloalkyle en C₅-C₈ non substitué ou substitué par halogéno, un radical aryle en C₆-C₁₀ non substitué ou substitué par halogéno ou un radical aralkyle en C₇-C₂₀ ramifié ou non ramifié, non substitué ou substitué par halogéno,
**caractérisée en ce qu'**elle ne contient aucun polymère à propriétés épaississantes.

2. Composition selon la revendication 1, **caractérisée en ce que** ladite une ou lesdites plusieurs substances du composant a) sont choisies dans le groupe constitué par le peroxyde d'hydrogène, le peroxyde d'urée, les perborates, les persulfates et des mélanges de ceux-ci.

3. Composition selon la revendication 1 ou 2, **caractérisée en ce que** la substance du composant a) est le peroxyde d'hydrogène et le peroxyde d'hydrogène est contenu dans la composition en des quantités de 0,5 à 20 % en poids, par rapport au poids total de la composition.

4. Composition selon un ou plusieurs des revendications 1 à 3, **caractérisée en ce que** ledit un ou lesdits plusieurs composés du composant d) se trouvent sous forme de l'acide ou sous forme de ses sels de métaux alcalins, de métaux alcalino-terreux ou d'amines ou sous forme de ses sels avec des ions opposés polymères.

5. Composition selon un ou plusieurs des revendications 1 à 4, **caractérisée en ce que** dans ledit un ou dans lesdits plusieurs composés de formule (I) ou dans leurs sels R1 est le groupe méthyle et R2 est le groupe cyclohexyle ou 2,4,4-triméthylpentyle.

6. Composition selon un ou plusieurs des revendications 1 à 5, **caractérisée en ce qu'**elle contient ladite une ou lesdites plusieurs substances du composant d) en des quantités de 0,1 ppm à 2 % en poids, par rapport au poids total de la composition.

7. Composition selon la revendication 6, **caractérisée en ce qu'**elle contient ladite une ou lesdites plusieurs substances du composant d) en des quantités de 0,5 à 100 ppm, par rapport au poids total de la composition.

8. Composition selon un ou plusieurs des revendications 1 à 7, **caractérisée en ce qu'**elle présente un pH de 2 à 11.

9. Composition selon la revendication 8, **caractérisée en ce qu'**elle présente un pH de 7 à 11.

10. Composition selon un ou plusieurs des revendications 1 à 9, **caractérisée en ce qu'**elle est une composition destinée à la décoloration et/ou à la teinture des cheveux ou une composition pour ondulation permanente.

11. Composition selon un ou plusieurs des revendications 1 à 9, **caractérisée en ce qu'**elle est une composition de produit de nettoyage oxydant.

12. Composition selon un ou plusieurs des revendications 1 à 9, **caractérisée en ce qu'**elle est une composition destinée au blanchiment oxydant de fibres ou de textiles.
